# EUROPÄISCHE PATENTANMELDUNG

(11) **EP 2 923 671 A1**
(43) Veröffentlichungstag der Anmeldung: **30.09.2015**
(21) Anmeldenummer: 15000752.4
(22) Anmeldetag: 13.03.2015
(51) Int. Cl.: A61F 2/00

(54) **IMPLANTIERBARES BAND**

(30) Priorität: 27.03.2014 AT 1392014
(71) Anmelder: A.M.I. Agency for Medical Innovations GmbH, 6800 Feldkirch (AT)
(72) Erfinder: Kociszewski, Jacek, 58332 Schwelm (DE)
(74) Vertreter: Hofmann, Ralf U.

(57) **Zusammenfassung**

Implantierbares Band zur Ausbildung einer alloplastischen Schlinge zur Behandlung von stressinduzierter Harninkontinenz bei Frauen, wobei das Band eine Vielzahl von Durchtrittsöffnungen aufweist, wobei das Band beidseitig eines Unterstützungsabschnitts (4) jeweils eine Verjüngung (7, 8) aufweist und eine jeweilige Verjüngung (7, 8) durch von den gegenüberliegenden Längsrändern (13, 14) des Bandes ausgehende Ausnehmungen (11, 12) ausgebildet wird und wobei das Band in den Bereichen seiner Verjüngungen (7, 8) eine geringere Breite (b) als im Unterstützungsabschnitt (4) und in an die Verjüngungen (7, 8) zu den Längsenden (5, 6) des Bandes hin anschließenden Halterungsabschnitten (9, 10) aufweist, wobei das Band über den Unterstützungsabschnitt (4), die Verjüngungen (7, 8) und die Halterungsabschnitte (9, 10) das gleiche Grundmaterial mit der gleichen Struktur aufweist.

## Beschreibung

Die Erfindung bezieht sich auf ein implantierbares Band zur Ausbildung einer alloplastischen Schlinge zur Behandlung von stressinduzierter Harninkontinenz bei Frauen, wobei das Band eine Vielzahl von Durchtrittsöffnungen aufweist.

Transvaginal platzierte alloplastische Schlingen, die mittels Bändern aus einem netzartigen Material gebildet werden, sind als Implantate zur Behandlung von stressinduzierter Harninkontinenz (=Stress- oder Belastungsinkontinenz) bei Frauen seit vielen Jahren bekannt. Bänder zur Ausbildung solcher Schlingen sind insbesondere aus monofilen Polypropylenfasern gewirkt und sind in verschiedenen Ausführungen auf dem Markt.

Diese Bänder sind in der Regel zwischen 8mm und 12mm breit und werden quer unterhalb der Harnröhre, zwischen Harnröhre und Vagina implantiert. Es wird eine Schlinge ausgebildet, deren beiden Enden entweder durch das Gewebe neben der Blase und hinter dem Schambein nach außen gezogen oder aber durch den Beckenknochen seitlich zur Vagina nach außen gezogen werden. Die Anlage der Schlinge erfolgt spannungsfrei, d.h. auf die Urethra soll kein Zug nach oben - zum Kopf hin - durch die implantierte Schlinge ausgeübt werden.

Obwohl spannungsfrei angelegt wird in bis zu 5% der Fälle eine Migration des Bandes (=Durchwanderung der Organwand) in die Urethra oder aber Vagina beobachtet, die einer operativen Nachbehandlung bedarf. In den meisten Fällen wird hierbei ein Längsrand des Bandes in der Vagina oder aber in der Harnröhre sichtbar.

Obwohl spannungsfrei angelegt, kann es durch die Anatomie des Patienten und/oder durch die gewählte Schlingenpassage zu Winkelabweichungen zwischen der Oberfläche der Urethra und Vagina einerseits und der Oberfläche des implantierten Bandes andererseits kommen. Solche Winkelabweichungen können dazu führen, dass die die Längskante des Bandes auf das darüber liegende Organ - die Harnröhre - oder auf das darunter liegende Organ - die Vagina - unerwünschten Druck ausübt, welcher in der weiteren Folge zum Durchwandern des Bandes im Bereich seines Längsrandes in das betroffene Organ führen kann.

Bei der Implantation eines solchen Bandes ist dieses üblicherweise zunächst von einer geschlossenen Kunststoffhülle umgeben. Nach dem Einbringen an den gewünschten Ort wird die Kunststoffhülle abgezogen. In diesem Zusammenhang wird auch auf die US 2003/0009181 A1 verwiesen.

Es sind auch Ausführungsformen solcher Bänder bekannt, die mit schleifenförmig verlaufenden Zugfäden versehen sind, durch welche die Lage und/oder Spannung postoperativ korrigiert werden kann. Nachoperationen können dadurch in schwierigen Fällen vermieden werden. Ein solches Band geht aus der US 8,016,743 B2 hervor.

Ein implantierbares Band zur Behandlung von Harninkontinenz der eingangs genannten Art geht aus der US 2013/0060078 A1 hervor. Es sind Abschnitte des Bandes vorgesehen, welche Verjüngungen des Bandes darstellen und eine Ausdehnung in Richtung der Längserstreckung des Bandes ermöglichen. Durch diese Dehnabschnitte wird eine visuelle Beurteilung und Einstellung der vom Band auf das zu unterstützende Gewebe ausgeübten Vorspannung ermöglicht. Zur dehnbaren Ausbildung der verjüngten Dehnabschnitte besitzen diese beispielsweise eine spezielle textile Struktur, die sich von derjenigen der daneben liegenden Abschnitte unterscheidet. Um die visuelle Beurteilung der Vorspannung des Bandes zu ermöglichen, liegen die Dehnabschnitte relativ weit von der Stelle entfernt, auf welcher die Harnröhre aufliegt.

Beim aus der US 2010/0261950 A1 bekannten implantierbaren Band zur Behandlung von Harninkontinenz sind Einstellabschnitte zur Veränderung der Vorspannung des Bandes vorgesehen. Diese können gegenüber daran anschließenden Abschnitten des Bandes verjüngt ausgebildet sein.

Aufgabe der Erfindung ist es ein vorteilhaftes Band der eingangs genannten Art bereitzustellen, bei dem die Gefahr einer Migration in die Urethra oder Vagina verhindert wird. Erfindungsgemäß gelingt dies durch ein Band mit den Merkmalen des Anspruchs 1.

Beim Band gemäß der Erfindung ist vorgesehen, dass beidseitig eines Unterstützungsabschnittes, welcher zur Unterstützung der Harnröhre im implantierten Zustand des Bandes vorgesehen ist, jeweils eine Verjüngung des Bandes vorhanden ist. Eine jeweilige Verjüngung wird hierbei durch Ausnehmungen ausgebildet, die von den gegenüberliegenden Längsrändern des Bandes ausgehen. Das Band weist damit in den Bereichen seiner Verjüngungen eine geringere Breite als im Unterstützungsabschnitt sowie eine geringere Breite als in Halterungsabschnitten auf, welche jeweils an die Verjüngungen in Richtung zu den Längsenden des Bandes hin anschließen. Das Band weist über den Unterstützungsabschnitt, die Verjüngungen und die Halterungsabschnitte das gleiche Grundmaterial mit der gleichen Struktur auf.

Durch die erfindungsgemäße Ausbildung wird in an die Längsränder des Bandes anschließenden Bereichen (=lateralen Bereichen) des Bandes eine größere Flexibilität in Hinblick auf Umbiegungen um gedachte Achsen erreicht, die zumindest annähernd parallel zur Richtung der Längserstreckung des Bandes liegen. Durch die Ausnehmungen kann im Bereich der Längsränder des Bandes kein Zug von den Halterungsabschnitten auf den Unterstützungsabschnitt übertragen werden. Gerade bei einer Winkelabweichung zwischen der Oberfläche der Urethra und der Vagina einerseits und der Oberfläche des implantierten Bandes andererseits kann sich somit der entsprechende laterale Bereich des Unterstützungsabschnittes des Bandes leichter an die Oberfläche der Urethra bzw. Vagina anpassen. Die Gefahr einer Migration des Bandes im Bereich seines Unterstützungsabschnittes in die Urethra oder Vagina wird dadurch wesentlich verringert.

Vorteilhafterweise beträgt die geringste Breite des Bandes im Bereich einer jeweiligen an den Unterstützungsabschnitt anschließenden Verjüngung weniger als zwei Drittel der Breite des Bandes im Unterstützungsabschnitt und weniger als zwei Drittel der Breite des Bandes im an die Verjüngung zum Längsende des Bandes hin anschließenden Halterungsabschnitt. Vorzugsweise weist das Band im Unterstützungsabschnitt und in den Halterungsabschnitten die gleiche Breite auf.

Das Band weist günstigerweise eine netzartige Struktur mit mehreren in Längsrichtung des Bandes verlaufenden Längssträngen auf, wobei die Längsstränge beispielsweise von Maschenstäbchen der durch Wirken ausgebildeten Struktur des Bandes gebildet werden. Von einer jeweiligen Ausnehmung, die im Anschluss an den Unterstützungsabschnitt von einem der Seitenränder des Bandes ausgehen, wird hierbei mindestens ein randseitiger Längsstrang unterbrochen.

Die auf die Richtung der Längserstreckung des Bandes bezogene Länge einer jeweiligen Verjüngung liegt günstigerweise im Bereich von 0,3 cm bis 4 cm, beispielsweise im Bereich von 0,3 cm bis 1,5 cm. Ein Wert im Bereich von 1 cm bis 3 cm ist besonders bevorzugt.

Vorteilhafterweise beträgt die auf die Richtung der Längserstreckung des Bandes bezogene Länge des Unterstützungsabschnitts, gemessen zwischen den Stellen, an denen die Ausnehmungen beidseitig des Unterstützungsabschnitts beginnen, 1 cm bis 3,5 cm, beispielsweise kann diese Länge im Bereich von 1,5 cm bis 3,5 cm liegen.

Weitere Vorteile und Einzelheiten der Erfindung werden im Folgenden anhand der beiliegenden Zeichnung erläutert. In dieser zeigen:
Fig. 1 ein Ausführungsbeispiel eines erfindungsgemäßen Bandes in Ansicht;
Fig. 2 einen schematischen Schnitt durch einen Abschnitt der Harnröhre und der oberen Wand der Vagina mit einem Abschnitt des implantierten Bandes (die Netzstruktur des Bandes ist in Fig. 2 der Einfachheit halber nicht dargestellt);
Fig. 3 ein weiteres Ausführungsbeispiel eines Bandes gemäß der Erfindung;
Fig. 4 einen vergrößerten Ausschnitt A des Bandes von Fig. 3.

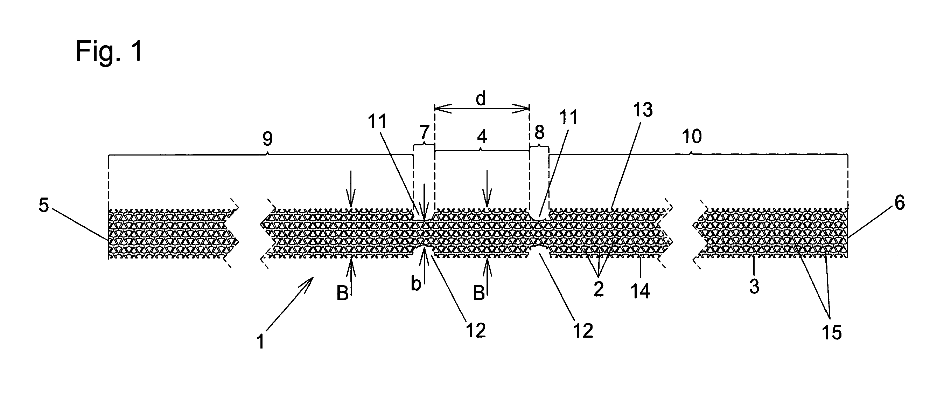

Ein erstes Ausführungsbeispiel eines erfindungsgemäßen Bandes ist in Fig. 1 dargestellt. Das Band 1 weist eine Vielzahl von Öffnungen 2 auf. Diese werden von einer netzartigen Struktur des Bandes gebildet. Zur Ausbildung einer netzartigen Struktur besteht das Band aus Fäden, von denen ein regelmäßiges Muster von Öffnungen freigelassen wird. Insbesondere ist das Band gewirkt, vorzugsweise in Form einer Kettenwirkware.

Die Öffnungen 2 ermöglichen in bekannter Weise, dass das Band von Gewebe durchwachsen wird, damit es im Körper nicht zu einer Abkapslung des Bandes kommt. Günstigerweise liegen die Öffnungsquerschnitte der Öffnungen im Bereich von 0,25mm² bis 9mm². Diese Öffnungen 2 werden auch als Makroporen des Bandes bezeichnet. Darüber hinaus weist das Band Mikroporen auf, die im Ausführungsbeispiel von Zwischenräumen zwischen den Fäden gebildet wird, aus denen das Band gewirkt ist. Diese weisen günstigerweise Öffnungsweiten von 50 µm bis 200 µm auf. Solche Mikroporen ermöglichen und beschleunigen ein Einwachsen von Fibroplasten.

Gewirkte Bänder zur Ausbildung von alloplastischen Schlingen zur Behandlung von stressinduzierter Harninkontinenz bei Frauen, welche eine solche netzartige Struktur aufweisen, sind bekannt.

Das Band 1 besitzt einen Unterstützungsabschnitt 4. In diesem Unterstützungsabschnitt 4 befindet sich das Band 1 im implantierten Zustand unterhalb der Urethra. Der Unterstützungsabschnitt 4 dient somit zur Unterstützung der Urethra.

Der Unterstützungsabschnitt 4 befindet sich in einem mittleren Drittel der Längserstreckung des Bandes. Vorzugsweise liegt die Längsmitte des Bandes in der Längsmitte des Unterstützungsabschnittes 4.

Zu den beiden Längsenden 5, 6 des Bandes hin schließt an den Unterstützungsabschnitt 4 jeweils eine Verjüngung 7, 8 des Bandes an. An eine jeweilige Verjüngung 7, 8 schließt zum jeweiligen Längsende 5, 6 des Bandes hin im Weiteren ein jeweiliger Halterungsabschnitt 9, 10 an. Im implantierten Zustand verlaufen die Halterungsabschnitte 9, 10 durch das Gewebe des Patienten und das Gewebe wächst in diese ein. Die Halterungsabschnitte 9, 10 dienen somit zur Halterung des Unterstützungsabschnittes 4.

Über die Längsausdehnung der jeweiligen Verjüngung 7, 8 ist die Breite b des Bandes kleiner als die Breite B des Bandes im Unterstützungsabschnitt im an die jeweilige Verjüngung zum jeweiligen Längsende 5, 6 des Bandes hin anschließenden Halterungsabschnitt 9, 10.

Die geringste Breite b des Bandes im Bereich einer jeweiligen Verjüngung 7, 8 beträgt im Ausführungsbeispiel weniger als zwei Drittel der Breite B des Bandes im Unterstützungsabschnitt 4 und weniger als zwei Drittel der Breite B des Bandes im an die Verjüngung 7, 8 zum Längsende 5, 6 des Bandes hin anschließenden Halterungsabschnitt 9, 10. Auch eine Ausbildung, bei welcher die geringste Breite b des Bandes im Bereich einer jeweiligen Verjüngung 7, 8 weniger als die Hälfte der Breite B des Bandes im Unterstützungsabschnitt 4 und weniger als die Hälfte der Breite B des Bandes im an die Verjüngung 7, 8 zum Längsende 5, 6 des Bandes hin anschließenden Halterungsabschnitt 9, 10 beträgt, ist denkbar und möglich.

Dadurch, dass die Halterungsabschnitte 9, 10 relativ breit ausgebildet sind, wird eine flach liegende Implantation der Halterungsabschnitte 9, 10 erleichtert.

Bevorzugterweise ist die Breite B des Bandes im Unterstützungsabschnitt 4 gleich wie die Breite B des Bandes im jeweiligen Halterungsabschnitt 9, 10.

Im Ausführungsbeispiel weist das Band über den Unterstützungsabschnitt 4 und über die an die Verjüngungen 7, 8 zu den Längsenden 5, 6 des Bandes hin anschließenden Halterungsabschnitte 9, 10 die gleiche, konstante Breite B auf. Auch eine über den Unterstützungsabschnitt 4 und/oder über die Halterungsabschnitte 9, 10 sich ändernde Breite ist denkbar und möglich.

Günstigerweise weisen der Unterstützungsabschnitt 4, die Verjüngungen 7, 8 und die Halterungsabschnitte 9, 10 das gleiche Grundmaterial des Bandes mit der gleichen Struktur auf.

Um die Unterstützungsfunktion der Harnröhre erfüllen zu können, ist das Band in Längsrichtung zumindest im Wesentlichen undehnbar ausgebildet.

Eine jeweilige Verjüngung 7, 8 wird durch zwei Ausnehmungen 11, 12 gebildet, die von den gegenüberliegenden Längsrändern 13, 14 des Bandes 1 an gegenüberliegenden Stellen ausgehen. Die Ausnehmungen 11, 12 begrenzen somit die Verjüngungen 7, 8 in die rechtwinkelig zur Richtung der Längserstreckung des Bandes stehende Richtung. Die Ausnehmungen 11, 12 sind im Ausführungsbeispiel U-förmig ausgebildet. Auch rechteckförmige oder V-förmige Ausbildungen sind denkbar und möglich.

Die Ausnehmungen 11, 12 können insbesondere durch Herausschneiden des Materials des ursprünglich mit gleicher Breite durchgehenden Bandes ausgehend von den Längsrändern 13, 14 des Bandes her ausgebildet werden. Solche schneidbaren Ausbildungen von gewirkten Bändern, ohne dass es zu einem Ausfransen an Seitenrändern der Ausnehmungen kommt, sind bekannt. Falls erforderlich, könnten die Seitenränder der Ausnehmungen 11, 12 auch durch Verschweißen oder Verkleben fixiert werden.

Die netzartige Struktur des Bandes weist mehrere, jeweils Öffnungen 2 des Bandes begrenzende Längsstränge 15 auf, die bezogen auf die Richtung der Längsausdehnung des Bandes durchgehend verlaufen. Bei der bevorzugten Ausbildung durch Wirken werden diese Längsstränge 15 von Maschenstäbchen gebildet.

Von einer jeweiligen Ausnehmung 11, 12 wird zumindest derjenige Längsstrang 15 unterbrochen, der am nächsten bei dem Längsrand 13, 14 liegt, von dem die Ausnehmung 11, 12 ausgeht. Eine jeweilige Ausnehmung 11, 12 könnte auch so tief sein, dass mindestens zwei randseitige Längsstränge 15 unterbrochen werden.

Durch die Unterbrechung des mindestens einen randseitigen Längsstrangs im Anschluss an die beiden Längsränder 13, 14 des Bandes 1 wird die Kraftübertragung über diese Längsstränge 15 von den Halterungsabschnitten 9, 10 auf den Unterstützungsabschnitt 4 unterbrochen. Der Unterstützungsabschnitt besitzt dadurch anschließend an die Längsränder 13, 14 des Bandes eine wesentlich größere Flexibilität gegenüber einer Biegung um eine gedachte, parallel zur Richtung der Längserstreckung des Bandes liegende Achse als ohne solche Ausnehmungen 11, 12.

Die Länge d des Unterstützungsabschnitts 4 in Richtung der Längserstreckung des Bandes 1 beträgt vorzugsweise 1,5 cm bis 3,5 cm, wobei diese Länge d zwischen den Stellen gemessen wird, an denen die Ausnehmungen 11, 12 beidseitig des Unterstützungsabschnitts 4 beginnen. Eine jeweilige Verjüngung 7, 8 erstreckt sich beispielsweise über 0,3 cm bis 1,5 cm in Richtung der Längserstreckung des Bandes.

Das Band 1 besteht beispielsweise aus Polypropylen, d.h. die, vorzugsweise monofilen, Fäden, aus welchen das Band gewirkt ist, bestehen aus Polypropylen.

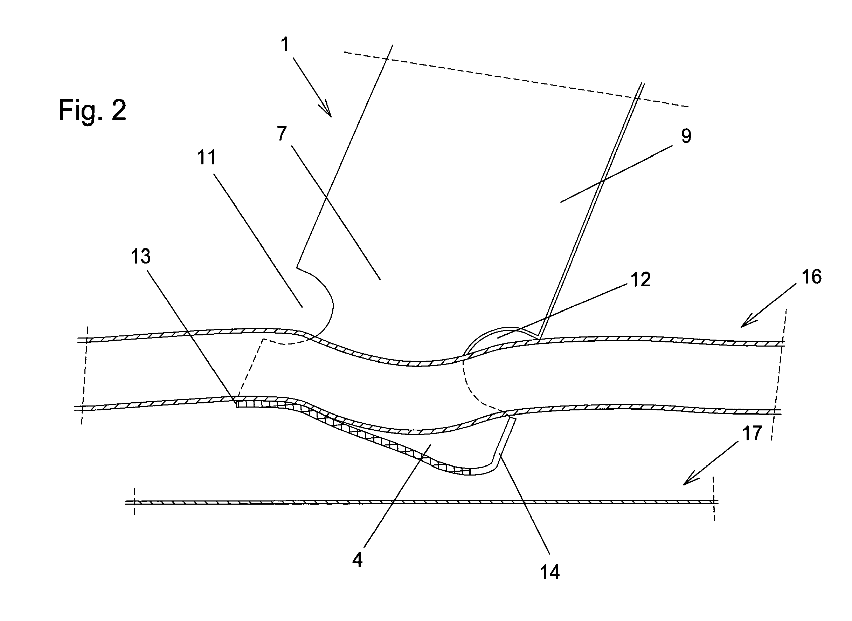

Ein Abschnitt des implantierten Bandes ist in Fig. 2 schematisch in einem durch den Unterstützungsabschnitt 4 verlaufenden Querschnitt dargestellt. Das Band 1 bildet im implantierten Zustand eine Art Schlinge, wobei es unter der Harnröhre 16 durchgeführt ist und mit dem Unterstützungsabschnitt 4 durch die Faszienbrücke zwischen der Urethra 16 und der Vagina 17 verläuft. Der Unterstützungsabschnitt 4 liegt somit über einem Bereich seiner Längserstreckung, vorzugsweise über einen mittleren Abschnitt seiner Längserstreckung, unterhalb der Urethra und stützt diese gegen eine Absenkung ab. Die Halterungsabschnitte 9, 10 sind durch das Gewebe des Patienten neben der Blase und hinter den Schambein nach oben gezogen oder aber durch den Beckenknochen seitlich zur Vagina nach außen gezogen.

Aus Fig. 2 ist ersichtlich, dass zumindest der in Fig. 2 sichtbare Halterungsabschnitt 9 eine Richtung seiner Längserstreckung aufweist, welche von einer Lage rechtwinkelig zur Längserstreckung der Urethra 16 bzw. Vagina 17 abweicht. Solche Abweichungen lassen sich bei der Implantation im Allgemeinen nicht vermeiden. Der Unterstützungsabschnitt 4 nimmt somit in dem Bereich, in dem er geschnitten dargestellt ist, zumindest in einem mittleren Abschnitt seiner Breitenerstreckung eine Ausrichtung an, die winkelig zur Richtung der Längserstreckung der Urethra 16 und Vagina 17 steht. Durch die Ausnehmungen 11, 12 wird aber eine so gute Flexibilität geschaffen, dass sich der Unterstützungsabschnitt 5 leicht an die Oberflächenstrukturen der Urethra 16 bzw. Vagina 17 anpassen kann. In der in Fig. 2 schematisch dargestellten Lage, ist der unterhalb der Urethra 16 liegende Bereich des Unterstützungsabschnittes 4, der an den höher liegenden Seitenrand 13 des Bandes anschließt, nach unten abgebogen und schmiegt sich an die Unterseite der Urethra 16 an. Einer Migration des Bandes 1 in die Urethra 16 wird dadurch entgegengewirkt.

Durch die Verjüngungen 7, 8 wird nicht nur eine bessere Flexibilität von an die Längsränder 13, 14 anschließenden Bereichen des Unterstützungsabschnitts 4 gegenüber Biegungen um parallel zur Richtung der Längserstreckung des Bandes 1 liegende Achsen, sondern auch eine insgesamte Winkelflexibilität des Unterstützungsabschnitts um eine parallel zur Längserstreckung des Bandes liegende Achse erreicht.

Ein insbesondere bei körperlichen Belastungen des Patienten auftretender Druck auf die oberhalb und unterhalb des Bandes 1 liegenden Organe 16, 17 durch die die Längsränder 13, 14 des Bandes bildenden Längskanten (=Schmalseiten) des Bandes 1 wird dadurch zumindest weitgehend vermieden.

Bei der Implantation des Bandes befindet sich dieses vorzugsweise zunächst in einer Hülle, welche nach dem Platzieren des Bandes abgezogen wird, wie dies bekannt ist.

Unterschiedliche Modifikationen des gezeigten Ausführungsbeispiels der Erfindung sind denkbar und möglich, ohne den Bereich der Erfindung zu verlassen. So könnte das Band beispielsweise mit schleifenförmig verlaufenden Zugfäden versehen sein, durch welche die Lage und/oder Spannung postoperativ korrigiert werden kann, wie dies beispielsweise aus dem eingangs genannten Stand der Technik gemäß der US 8,016,743 B2 hervorgeht.

Anstelle durch Wirken könnte ein eine Vielzahl von Durchtrittsöffnungen aufweisendes Band beispielsweise auch in Form eines gelochten folien- oder blattförmigen Materials ausgebildet sein, um Makroporen zu bilden. Mikroporen könnten dann beispielsweise durch Laserbohrungen oder durch die Porosität des Materials gebildet werden.

Das Band könnte auch aus anderen langzeitimplantattauglichen, also insbesondere bioinerten, Materialien ausgebildet sein, z.B. Polyester, Nylon oder Silikon. Zur Verringerung der Längsdehnbarkeit könnte das Band auch eingebettete Zugfasern aufweisen.

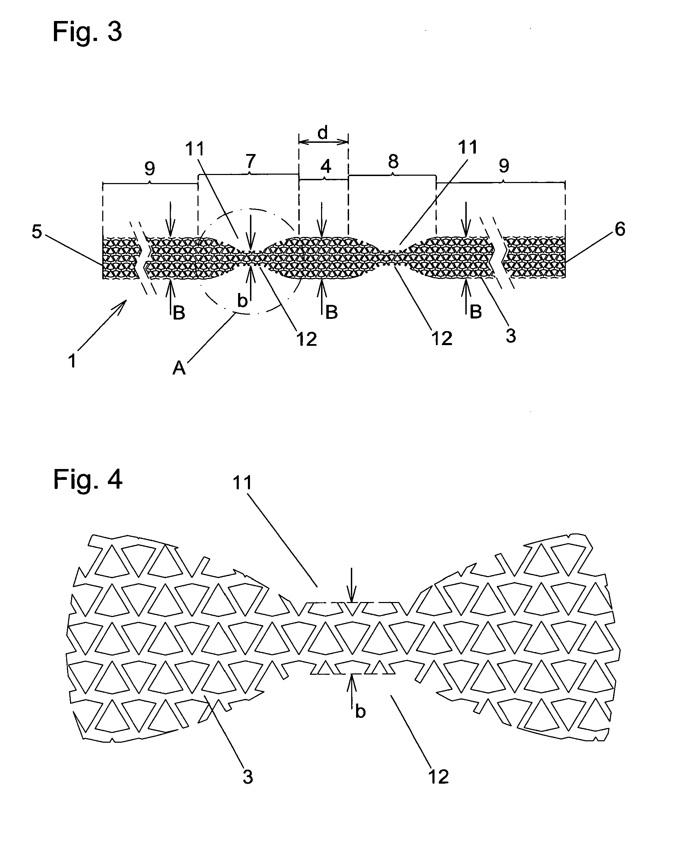

Ein zweites Ausführungsbeispiel eines erfindungsgemäßen Bandes 1 ist in Fig. 3 und 4 dargestellt. Abgesehen von den im Folgenden beschriebenen Unterschieden entspricht die Ausbildung derjenigen des ersten Ausführungsbeispiels und es wird entsprechend auf die Beschreibung des ersten Ausführungsbeispiels verwiesen. Auch die im Zusammenhang mit dem ersten Ausführungsbeispiel beschriebenen möglichen Modifikationen sind möglich.

Der Unterschied des zweiten Ausführungsbeispiels im Vergleich zum ersten Ausführungsbeispiel besteht vor allem darin, dass die Verjüngungen 7, 8 des Bandes 1 sowohl zum Unterstützungsabschnitt 4 als auch zu den Halterungsabschnitten 9, 10 hin abgerundet auslaufen. Die Verjüngungen 7, 8 weisen hierbei auch eine größere Ausdehnung in Längserstreckung des Bandes 1 als im ersten Ausführungsbeispiel auf und ihre Längen können beispielsweise im Bereich von 1 cm bis 3 cm liegen.

Die Länge d des Unterstützungsabschnitts 4 kann beispielsweise im Bereich von 1 cm bis 2 cm liegen.

Die geringste Breite b des Bandes 1 im Bereich einer jeweiligen Verjüngung 7, 8 ist hier kleiner als die Hälfte der Breite B des Bandes 1 im Unterstützungsabschnitt 4 und kleiner als die Breite B des Bandes 1 im an die jeweilige Verjüngung 7, 8 zum Längsende 5, 6 des Bandes 1 hin anschließenden Halterungsabschnitt 9, 10.

Bei einer derartigen Ausbildung des Bandes 1 hat sich als besonders vorteilhaft erwiesen, dass Verletzungen des Gewebes vermieden werden, an welchem das Band 1 im Bereich der Verjüngungen 7, 8 anliegt.

Im Bereich einer jeweiligen Verjüngung 7, 8 ist das implantierte Band 1 durch eine in einer Faszie ausgebildeten Öffnung durchgezogen. Durch die relativ lange Ausbildung der Verjüngungen 7, 8 und ihre relativ geringe Breite b, kann einem Zusammenfalten des Bandes 1 parallel zur Längsrichtung im Bereich der Öffnung durch die Faszie und somit auch im Bereich der an die jeweilige Verjüngung 7, 8 anschließenden Abschnitte des Bandes 1 entgegengewirkt werden.

Die Verjüngungen 7, 8 werden günstigerweise durch Abschmelzen von Material des Bandes 1 ausgebildet. Es kann hierzu vorteilhafterweise ein Lötkolben herangezogen werden, wobei auch ein Laser einsetzbar ist. Durch das Abschmelzen erfolgt auch eine abgerundete Ausbildung der Seitenränder des Bandes 1 im Bereich der Verjüngungen 7, 8. Eventuell zurückbleibende abstehende Enden der netzartigen Struktur des Bandes 1 werden hierbei ebenfalls abgerundet und gegebenenfalls mit benachbarten abstehenden Enden verschmolzen. Damit kann ein Ausfransen der Seitenränder der Ausnehmungen 11, 12 verhindert werden. Dies ist in den Figuren der Einfachheit halber nicht dargestellt.

Auch beim ersten Ausführungsbeispiel erfolgt beim Ausbilden der Verjüngungen 7, 8 durch Abschmelzen eine solche Abrundung der zurückbleibenden abstehenden Enden der netzartigen Struktur des Bandes 1.

### Legende zu den Hinweisziffern:

- 1: Band
- 2: Öffnung
- 3: Faden
- 4: Unterstützungsabschnitt
- 5: Längsende
- 6: Längsende
- 7: Verjüngung
- 8: Verjüngung
- 9: Halterungsabschnitt
- 10: Halterungsabschnitt
- 11: Ausnehmung
- 12: Ausnehmung
- 13: Längsrand
- 14: Längsrand
- 15: Längsstrang
- 16: Urethra
- 17: Vagina

## Patentansprüche

1. Implantierbares Band zur Ausbildung einer alloplastischen Schlinge zur Behandlung von stressinduzierter Harninkontinenz bei Frauen, wobei das Band eine Vielzahl von Durchtrittsöffnungen aufweist, wobei das Band beidseitig eines Unterstützungsabschnitts (4) jeweils eine Verjüngung (7, 8) aufweistund eine jeweilige Verjüngung (7, 8) durch von den gegenüberliegenden Längsrändern (13, 14) des Bandes ausgehende Ausnehmungen (11, 12) ausgebildet wird und wobei das Band in den Bereichen seiner Verjüngungen (7, 8) eine geringere Breite (b) als im Unterstützungsabschnitt (4) und in an die Verjüngungen (7, 8) zu den Längsenden (5, 6) des Bandes hin anschließenden Halterungsabschnitten (9, 10) aufweist, **dadurch gekennzeichnet, dass** das Band über den Unterstützungsabschnitt (4), die Verjüngungen (7, 8) und die Halterungsabschnitte (9, 10) das gleiche Grundmaterial mit der gleichen Struktur aufweist.

2. Band nach Anspruch 1, **dadurch gekennzeichnet, dass** die geringste Breite (b) des Bandes im Bereich einer jeweiligen an den Unterstützungsabschnitt (4) anschließenden Verjüngung weniger als zwei Drittel, vorzugsweise weniger als die Hälfte, der Breite (B) des Bandes im Unterstützungsabschnitt (4) und weniger als zwei Drittel, vorzugsweise weniger als die Hälfte, der Breite (B) des Bandes im an die Verjüngung (7, 8) zum Längsende (5, 6) des Bandes hin anschließenden Halterungsabschnitt (9, 10) beträgt.

3. Band nach Anspruch 1 oder 2, **dadurch gekennzeichnet, dass** die Breite (B) des Bandes im Unterstützungsabschnitt (4) und in den an die Verjüngungen (7, 8) zu den Längsenden (5, 6) des Bandes hin anschließenden Halterungsabschnitten (9, 10) 8mm bis 15mm beträgt.

4. Band nach einem der Ansprüche 1 bis 3, **dadurch gekennzeichnet, dass** die geringste Breite (b) des Bandes im Bereich einer jeweiligen Verjüngung (7, 8) 1,5mm bis 5mm beträgt.

5. Band nach einem der Ansprüche 1 bis 4, **dadurch gekennzeichnet, dass** die Breite (B) des Bandes über die Längserstreckung des Unterstützungsabschnitts (4) konstant ist und dass die Breite (B) des Bandes über die Längserstreckungen der an die Verjüngungen (7, 8) zu den Längsenden (5, 6) des Bandes hin anschließenden Halterungsabschnitten (9, 10) konstant ist.

6. Band nach einem der Ansprüche 1 bis 5, **dadurch gekennzeichnet, dass** das Band über den Unterstützungsabschnitt (4) und über die an die Verjüngungen (7, 8) zu den Längsenden (5, 6) des Bandes hin anschließenden Halterungsabschnitte (9, 10) die gleiche, konstante Breite (B) aufweist.

7. Band nach einem der Ansprüche 1 bis 6, **dadurch gekennzeichnet, dass** das Band eine netzartige Struktur aufweist.

8. Band nach Anspruch 7, **dadurch gekennzeichnet, dass** die netzartige Struktur des Bandes mehrere in Richtung der Längserstreckung des Bandes durchgehende Längsstränge (15) aufweist, wobei von einer jeweiligen Ausnehmung (11, 12) mindestens ein randseitiger Längsstrang (15) unterbrochen ist.

9. Band nach einem der Anspruch 7 oder 8, **dadurch gekennzeichnet, dass** das Band gewirkt ist.

10. Band nach einem der Ansprüche 1 bis 9, **dadurch gekennzeichnet, dass** das Band aus Polypropylen besteht.

11. Band nach einem der Ansprüche 1 bis 10, **dadurch gekennzeichnet, dass** die Länge (d) des Unterstützungsabschnitts (4) in Richtung der Längserstreckung des Bandes, gemessen zwischen den Stellen, an denen die Ausnehmungen (11, 12) beidseitig des Unterstützungsabschnitts (4) beginnen, 1 cm bis 3,5 cm, vorzugsweise 1,5 cm bis 3,5 cm, beträgt.

12. Band nach einem der Ansprüche 1 bis 11, **dadurch gekennzeichnet, dass** die Länge einer jeweiligen Verjüngung (7, 8) in Richtung der Längserstreckung des Bandes 0,3 cm bis 4 cm, vorzugsweise 0,3 cm bis 1,5 cm, beträgt.

13. Band nach einem der Ansprüche 1 bis 11, **dadurch gekennzeichnet, dass** die Länge einer jeweiligen Verjüngung (7, 8) in Richtung der Längserstreckung des Bandes 1 cm bis 3 cm beträgt.

14. Band nach einem der Ansprüche 1 bis 13, **dadurch gekennzeichnet, dass** die Ausnehmungen (11, 12) U-förmig, V-förmig oder rechteckförmig ausgebildet sind.

15. Band nach einem der Ansprüche 1 bis 14, **dadurch gekennzeichnet, dass** sich der Unterstützungsabschnitt (4) im mittleren Drittel der Längserstreckung des Bandes befindet.
